# EUROPÄISCHE PATENTANMELDUNG

(11) **EP 2 404 549 A1**
(43) Veröffentlichungstag der Anmeldung: **11.01.2012**
(21) Anmeldenummer: 11005595.1
(22) Anmeldetag: 08.07.2011
(51) Int. Cl.: A61B 5/022, A61B 17/135, A61B 5/0225

(54) **Gerät zum Stauen von Blut in einer Vene**

(30) Priorität: 08.07.2010 DE 102010026576
(71) Anmelder: Brunn-Schulte-Wissing, Peter, 48432 Rheine (DE); Linek, Rudolf, 38518 Gifhorn (DE)
(72) Erfinder: Brunn-Schulte-Wissing, Peter, 48432 Rheine (DE); Linek, Rudolf, 38518 Gifhorn (DE)
(74) Vertreter: Ahrens, Thomas

(57) **Zusammenfassung**

Die vorliegende Erfindung betrifft ein Gerät zum Stauen von Blut in einer Vene.

Dabei wird mittels einer Kompressionskammer (20) ein solcher Druck auf einen Körperteil ausgeübt, dass durch eine darin befindliche Arterie Blut fließen kann während eine Vene derart abgeklemmt ist, dass kein Blut durch sie durchfließt. Damit staut sich das Blut in der Vene, die daraufhin ihr Volumen vergrößert.

Das Besondere an der Erfindung ist, dass ein Sensor vorgesehen ist, der diesen Zustand detektieren und ein zugehöriges Signal abgeben kann.

Die Erfindung lässt sich insbesondere dazu nutzen, eine Venenpunktierung zu erleichtern.

## Beschreibung

Die vorliegende Erfindung betrifft ein Gerät zum Stauen von Blut in einer Vene, insbesondere innerhalb einer Extremität, wie Arm oder Bein, bei einem Menschen.

Das Herz-Kreislauf-System eines Lebewesens, wie eines Menschen oder eines Wirbeltieres, umfasst Arterien und Venen. Durch die Arterien wird Blut vom Herzen in einen Arm - oder eine sonstige Extremität - hineingepumpt, um die dort vorhandenen Körperzellen zu versorgen. Durch die Venen fließt das verbrauchte Blut zurück in den Rumpf. Die Venen liegen im äußeren Bereich des Armes und die Arterien befinden sich weiter im Inneren davon.

Für eine Blutentnahme bei einer zu behandelnden Person wird üblicherweise eine Vene in einem Arm, bevorzugt in einem Unterarm, punktiert. Um diesen Vorgang zu erleichtern, wird die Vene im Oberarm gestaut. Dafür wird üblicherweise ein Stauschlauch - auch ein Band oder dergleichen - benutzt. Dieser wird um den Oberarm der Person geschlungen und zugezogen, wodurch die Venen abgedrückt werden und das darin befindliche Blut gestaut wird. Dieses bewirkt ein Hervorquellen der Venen, wodurch eine behandelnde Person - wie ein Arzt, ein Sanitäter oder ähnlich geschulte Personen - auf einfache Weise eine Kanüle zur Blutentnahme einstechen und somit Blut entnehmen kann.

Diese Methode hat jedoch den Nachteil, dass die behandelnde Person keine Kontrolle darüber hat, mit welchem Druck der Stauschlauch auf den Oberarm in Abhängigkeit von der Zugkraft einwirkt. Dadurch kommt es häufig vor, dass nicht nur die Venen sondern auch die tiefer liegenden Arterien der zu behandelnden Person abgedrückt werden. Damit wird der Effekt der Überfüllung der Venen gar nicht oder nur unzureichend bewirkt.

Es ist auch bekannt, bei einem üblichen Verfahren zur Blutdruckmessung um den Oberarm einer zu behandelnden Person eine Druckmanschette zu legen und diese solange mit Druck zu beaufschlagen, bis die Strömung sowohl in der Vene als auch in der Arterie gesperrt wird. Anschließend wird der Druck innerhalb der Manschette langsam entspannt, solange bis in der Arterie erneut eine Blutströmung entsteht. Der hierdurch gemessene Druck entspricht dem systolischen Druck. Durch die Öffnung der Arterie bei noch geschlossener Vene kommt es zu Blutverwirbelungen, wodurch Geräusche - so genannte Korotkow-Geräusche - entstehen, die von einer behandelnden Person, wie ein Arzt oder dergleichen, mittels eines Stethoskops wahrgenommen werden können. Bei weiterem Druckentspannen der Manschette beginnt auch in der Vene zu einem bestimmten Zeitpunkt erneut die Blutströmung, wobei der dann vorherrschende Druck den diastolischen Druck darstellt. Dann fließt das Blut verwirbelungsfrei und damit nahezu geräuschlos durch die Blutgefäße.

Entsprechende Manschetten unterschiedlicher Größe sind beispielsweise bekannt aus DE 197 51 564 A1.

Aus DE 100 07 231 A1 ist ein Venenstaugerät bekannt, das den Effekt der Überfüllung der Venen sicherstellen soll. Dabei handelt es sich um eine Vorrichtung mit einer Druckschlauchmanschette, an die eine Druckquelle - wie eine Druckpumpe - angeschlossen ist. Außerdem ist ein Druckeinstell-Hilfsmittel - wie ein an einem Mikrocomputer angeschlossener Drucksensor oder ein Überdruckventil - vorhanden, mittels welchem der Manschetteninnendruck erfasst und/oder auf einen vorbestimmten oder vorbestimmbaren Wert eingestellt werden kann. Dieser Wert soll einem Druck entsprechen, der ein Abdrücken einer Vene bewirkt, gleichzeitig jedoch den Strömungsquerschnitt einer Arterie zumindest teilweise erhält.

Der vorbestimmte oder vorbestimmbare Druckwert kann nach der genannten Schrift auf verschiedene Weise ermittelt werden. So soll bei jüngeren Menschen der systolische Druck innerhalb der Arterie bei einem Wert von ca. 100 bis 120 mm HG und der diastolische Druck innerhalb der Vene bei einem Wert von ca. 60 bis 70 mm HG liegen. Bei älteren Menschen steigt der systolische Druck auf über 120 mm HG, während der diastolische Druck ca. 90 mm HG beträgt. Daraus ergibt sich ein Druckfenster von ca. 30 bis 40 mm HG, jedoch auf unterschiedlichem Niveau. Es hat sich gezeigt, dass sich laut der genannten Schrift dieses Druckfenster altersbedingt bei jungen und alten Menschen im Wesentlichen gleichmäßig unterscheidet, so dass es ausreichen soll, zumindest zwei Druckwerte bezogen auf die verwendete Druckschlauchmanschette für junge und alte zu behandelnde Personen zu bestimmen, welche in einer Steuervorrichtung abgespeichert werden können. Dieses Druckfenster ist demnach groß genug, um individuelle Abweichungen, beispielsweise im Falle von vorliegenden Krankheiten oder unterschiedlichen körperlichen Konstitutionen auszugleichen. Somit sollen nach der dort vorgestellten Lehre zumindest zwei Werte in Abhängigkeit der Ausgestaltung der Druckschlauchmannschette abgespeichert werden, die zwei Drücken entsprechen welche auf die Vene und die Arterie jeweils eine Druckkraft zwischen 60 und 120 vorzugsweise 80 und 100 mm HG ausüben. Alternativ wird dort auch vorgeschlagen, den systolischen und den diastolischen Druck für jede zu behandelnde Person individuell zu messen und anschließend einen Mittelwert davon zu bestimmen, der den vorbestimmbaren Wert darstellt.

Das dort vorgestellte Venenstaugerät funktioniert derart, dass nach dem Anlegen der Druckschlauchmanschette die Druckquelle gestartet wird. Darauf steigt der Druck innerhalb der Manschette. Der entsprechende Wert wird registriert und bei Erreichen des vorbestimmten Druckwertes wird die Druckquelle abgeschaltet.

Das in DE 100 07 231 A1 vorgestellte Venenstaugerät benötigt somit stets einen Druckwert, der bestimmt werden kann aufgrund von Erfahrungswerten oder einer vorangegangenen Messung. Damit ist eine Unsicherheit vorhanden, die dadurch verursacht wird, dass bei der zu behandelnden Person im Zeitpunkt der Venenpunktierung andere Blutdruckverhältnisse herrschen können als zuvor vermutet oder gemessen.

Es ist somit die Aufgabe der vorliegenden Erfindung, ein Gerät zum Stauen einer Vene zu ermöglichen, das unmittelbar im Zeitpunkt der Venenpunktierung einen derartigen Druck auf eine Extremität ausübt, dass die Arterien im Wesentlichen geöffnet und die Venen nahezu verschlossen sind.

Diese Aufgabe wird gelöst durch die Merkmale des Hauptanspruchs, wobei in den Unteransprüchen vorteilhafte Weiterbildungen angegeben sind.

Das erfindungsgemäße Gerät zum Stauen von Blut in einer Vene weist eine Manschette auf, die geeignet ist, an einem geeigneten Körperteil eines Menschen oder eines Wirbeltieres befestigt zu werden. Bevorzugterweise ist diese Manschette ähnlich gestaltet wie eine Druckmanschette, die üblicherweise auch verwendet wird zur Messung des Blutdrucks. Der geeignete Körperteil, wie ein Arm oder ein Bein, ist ein solches, welches mindestens eine Arterie und mindestens eine Vene enthält.

Die Manschette enthält eine Kompressionskammer, die geeignet ist, Druck auf den Körperteil derart auszuüben, so dass sowohl die Vene als auch die Arterie verschlossen werden und dadurch ein Blutfluss blockiert wird. Eine solche Kompressionskammer kann als luftdichte Kammer gestaltet sein, in die mittels einer Pumpe, eines Blasebalgs oder dergleichen Gas, wie beispielsweise Luft, oder Flüssigkeit zugeführt werden kann und die dabei ihr Volumen vergrößert. Durch geeignete Ansteuerung einer solchen Pumpe oder entsprechende Betätigung des Blasebalgs kann die Volumenvergrößerung gesteuert werden. Eine weitere Möglichkeit, die Volumenvergrößerung zu steuern besteht zusätzlich oder stattdessen darin, Gas bzw. Flüssigkeit gezielt ablassen zu können. Das kann beispielsweise mittels eines elektrisch ansteuerbaren Ventils oder eines manuell steuerbaren Ablassventils erfolgen, wie es auch bei der Blutdruckmessung verwendet wird.

Die Kompressionskammer kann stattdessen oder zusätzlich auch Mittel enthalten, die auf andere Weise Druck auf den Körperteil ausüben, wie mittels eines Elektromagneten, eines schwenkbaren Teils, das durch elektro-mechanische oder pneumatische Ansteuerung bewegt werden kann, oder dergleichen.

Das erfindungsgemäße Gerät weist ebenfalls einen Sensor auf, der geeignet ist, einen Blutfluss durch die Arterie zu messen und der ein entsprechendes Signal abgibt, das beispielsweise von einer Anzeigeeinheit und/oder einer Steuereinheit verarbeitet werden kann.

Ein geeigneter Sensor kann beispielsweise als Drucksensor ausgebildet sein, der detektiert, wann ein bestimmter Druck in der Vene herrscht. Die bei der Venenstauung auftretende Ausdehnung der Vene kann auch mittels eines Dehnungssensors erfasst werden. Denkbar ist auch ein akustischen Sensor, ähnlich wie ein Mikrofon. Dieser kann beispielsweise das so genannte Korotkow-Geräusch aufnehmen, das üblicherweise bei einer Blutdruckmessung mit einem Stethoskop hörbar ist.

Eine geeignete Anzeigeeinheit kann optische und/oder akustische Signale in Abhängigkeit vom Wert des Sensorsignals erzeugen. Dabei ist es insbesondere denkbar, dass genau dann, wenn der Sensor einen Venenstau detektiert, d.h. einen Blutfluss durch die Arterie ohne einen solchen durch die Vene, eine Lampe, wie beispielsweise LED oder dergleichen, aufleuchtet und/oder ein passendes akustisches Signal ertönt. Es ist weiterhin denkbar, dass die Anzeigeeinheit einen Bildschirm und/oder eine Sprachausgabe umfasst, über die ausführliche Informationen über den Betriebszustand des erfindungsgemäßen Gerätes an einen Nutzer abgegeben werden können.

Mittels einer elektronischen Steuerung kann ein ganz bestimmtes Messverfahren umgesetzt werden. Dadurch werden ein hoher Bedienkomfort und eine hohe Zuverlässigkeit bei dem erfindungsgemäßen Gerät ermöglicht. Ein bevorzugtes Verfahren läuft derart ab, dass durch Ansteuerung einer Pumpe oder eines sonstigen geeigneten Mittels die Kompressionskammer zunächst den Druck sowohl auf die Vene als auch auf die Arterie so lange erhöht, bis kein Blut mehr fließt. In einem nächsten Schritt wird der Druck vermindert, beispielweise durch Ablassen von Gas oder Flüssigkeit oder aber durch entsprechende Ansteuerung anderer oben genannter Mittel. Dadurch wird der Blutfluss in der Arterie möglich, wobei die Vene jedoch noch vollständig oder fast vollständig verschlossen ist. Dieser Zustand wird mittels des Sensors detektiert und es wird ein entsprechendes Signal über die Anzeigeeinheit abgegeben. Außerdem wird durch geeignete Ansteuerung der Kompressionskammer sichergestellt, dass dieser Zustand lange genug aufrecht erhalten wird. Das Ende davon kann entweder durch eine Zeitsteuerung oder durch eine entsprechende Eingabe eines Nutzers bewirkt werden.

Das erfindungsgemäße Gerät erlaubt es, einen Zustand zu bestimmen, bei dem durch die Arterie Blut strömt und die zu gehörige Vene blockiert bzw. abgeklemmt ist, so dass durch sie gar kein oder fast kein Blut fließen kann. Dadurch wird die Vene gestaut und ist einfach zu punktieren.

Die Erfindung unterscheidet sich von dem bekannten Stand der Technik also insbesondere dadurch, dass der genannte Zustand (Arterie offen, Vene quasi geschlossen) direkt gemessen und nicht indirekt anhand von bestimmten Blutdruckwerten ermittelt wird.

Im Folgenden werden weitere Einzelheiten und Vorteile der Erfindung anhand bevorzugter Ausführungsbeispiele erläutert. Dabei zeigen:
- Fig. 1: Die Anordnung einer Manschette um einen Arm
- Fig. 2: Ein Blockschaltbild mit elektronischer Steuerung
- Fig. 3: Ein Blockschaltbild ohne elektronische Steuerung.

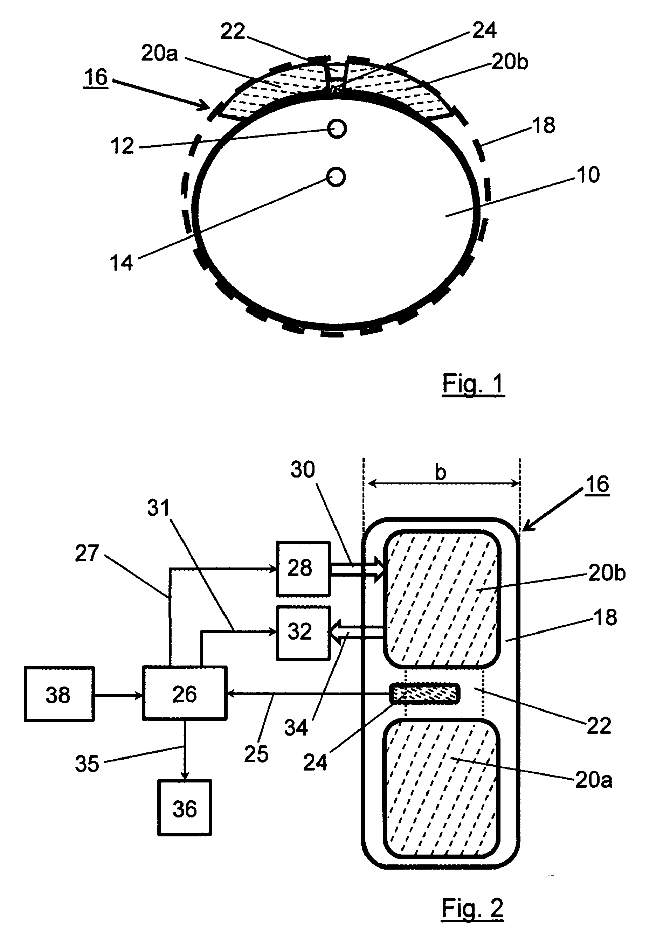

Fig. 1 zeigt symbolisch und stark vereinfacht als Querschnittsansicht einen Arm 10, konkret einen Oberarm, in dem sich eine Vene 12 und eine Arterie 14 befinden. Über die Arterie 14 fließt üblicherweise frisches Blut vom Rumpf in den unteren Teil des Arms 10 hinein und aus der Vene 12 wird es zurück in den Rumpf in Richtung Herz geleitet. Um den Arm 10 herum ist eine Manschette 16 gelegt. Diese weist in ihrem Äußeren ein Kompressionsband 18 auf. Zwischen diesem und dem Arm 10 befinden sich eine erste Luftkammer 20a und eine zweite Luftkammer 20b. Beide Luftkammern 20 sind mittels eines Übergangstücks 22 miteinander verbunden, so dass ein Druckausgleich zwischen den Luftkammern 20 ermöglicht wird. Unterhalb des Kompressionsbands 18 und in Kontakt mit dem Arm 10 befindet sich ein Sensor 24. Dieser ist so gestaltet, dass er den Blutfluss in der Vene 12 und in der Arterie 14 detektieren kann. Das kann z.B. erfolgen durch Messung des Pulses, der Pulsfrequenz und/oder des Blutdrucks.

Fig. 2 zeigt symbolisch als Draufsicht den oberen Abschnitt der Unterseite der Manschette 16, also der Seite, die in Fig. 1 in Richtung zu dem Arm 10 zeigt. Der obere Abschnitt ist derjenige, der in Fig. 1 oberhalb des Armes 10 dargestellt ist. Dabei ist u.a. gut erkennbar, dass das Kompressionsband 18 eine Breite b hat, deren Wert in dem Ausführungsbeispiel bevorzugterweise bei etwa 5 cm liegt.

Der Übergangskanal 22, der die beiden Luftkammern 20 miteinander verbindet, ist durch gestrichelte Linien angedeutet. Der Sensor 24 ist mit einem elektronischen Steuergerät 26 verbunden und kann so seine Sensorsignale 25 abgeben. An das Steuergerät 26 ist außerdem eine Pumpe 28 angeschlossen, die durch ein erstes Steuersignal 27 ansteuerbar ist. Diese kann im Betrieb Luft vom Außenbereich über einen ersten Luftschlauch 30 in die Luftkammer 20b pumpen. Über den Übergangskanal 22 gelangt die eingepumpte Luft ebenfalls in die Luftkammer 20a. Das Steuergerät 26 kann außerdem ein Ventil 32 mittels eines zweiten Steuersignals 31 ansteuern, das über einen zweiten Luftschlauch 34 Luft aus der Luftkammer 20b in den Außenbereich ablassen kann. Wenn das geschieht, ist über den Übergangskanal 22 auch die Luftkammer 20a davon betroffen. Das Steuergerät 26 ist außerdem mit einer Anzeigeeinheit 36 verbunden, die bei entsprechender Ansteuerung durch das Anzeigesignal 35 optische und/oder akustische Signale abgeben kann. Weiterhin ist eine Eingabestufe 38 vorhanden, über die die Funktion des Steuergerätes 26 gestartet werden kann.

Die in den Fig. 1 und 2 dargestellte Vorrichtung funktioniert folgendermaßen. Vor Inbetriebnahme der Vorrichtung wird die Manschette 16 mit nur leichtem Druck um den Arm 10 gelegt und mittels des Kompressionsbandes 18 fixiert, das einen passenden Verschluss (nicht gezeigt), wie beispielsweise einen Klettverschluss, aufweist. Dabei ist das Ventil 32 geöffnet, so dass Luft aus den Luftkammern 20 entweichen kann. Dadurch herrscht, verglichen mit dem Außenbereich, also der Umgebung, kein Überdruck und die Luftkammern 20 sind entspannt. Der Blutfluss sowohl in der Vene 12 als auch in der Arterie 14 wird von außen nicht beeinträchtigt. Nach Betätigung der Eingabeeinheit 38 wird die Funktion des Steuergeräts 26 gestartet, das daraufhin das Ventil 32 derart ansteuert, dass es geschlossen ist und keine Luft mehr aus den Luftkammern 20 entweichen kann. In einem weiteren Schritt wird die Pumpe 28 angesteuert. Dadurch wird Luft in die Luftkammern 20 gepumpt, so dass in ihnen ein Überdruck entsteht und sie sich ausdehnen. Diese Ausdehnung erfolgt aufgrund der Struktur und Beschaffenheit des Kompressionsbandes 18 im Wesentlichen nach innen, also in Richtung des Arms 10. Mit zunehmendem Druck und somit zunehmender Ausdehnung der Luftkammern 20 wird zunächst die Vene 12 und anschließend die Arterie 14 abgeklemmt. Dadurch ist der Blutfluss unterbrochen. Das wird von dem Sensor 24 detektiert, der ein entsprechendes Signal 25 an das Steuergerät 26 gibt. Dieses schaltet die Pumpe 28 ab und nahezu gleichzeitig oder etwas später wird das Ventil 32 derart angesteuert, dass es öffnet und so zeitlich gesteuert Luft aus den Luftkammern 20 entweichen lässt. Diese entspannen, so dass der Druck mehr oder weniger langsam auf den Arm 10 und somit auf die Vene 12 und die Arterie 14 abnimmt. Auf die Arterie 14 wird ein geringerer Druck ausgeübt als auf die Vene 12, da erstere weiter von der Oberfläche des Arms 10 und somit von den Luftkammern 20 entfernt liegt. Somit wird ein Blutfluss zunächst durch die Arterie 14 ermöglicht. Das wird mittels des Sensors 24 detektiert, beispielsweise anhand eines Puls-Signal-Klopfens, und dem Steuergerät 26 signalisiert. Das steuert daraufhin das Ventil 32 derart an, dass es schließt und keine weitere Luft aus den Luftkammern entweichen kann. Der Druck in den Luftkammern und deren Ausdehnung bleiben also zunächst konstant und zwar derart, dass die Vene 12 geschlossen und die Arterie 14 geöffnet bleiben. Damit entsteht ein Staudruck in der Vene 12, so dass sie dadurch geweitet wird. Dieser Zustand ist gut geeignet, um die Vene 12 zu punktieren und so beispielsweise Blut zu entnehmen oder Infusionen, wie Medikamente oder dergleichen, zuzuführen. Damit dieser Zustand geeignet genutzt werden kann, wird die Anzeigeeinheit 36 angesteuert, die daraufhin ein akustisches und/oder optisches Signal abgibt.

Dieser Zustand wird bevorzugterweise dadurch beendet, dass der Nutzer über die Eingabeeinheit 38 ein entsprechendes Signal gibt. Dann wird das Ventil 32 geöffnet, so dass die Luftkammern 20 entleert werden und der Blutfluss sowohl durch die Arterie 14 als auch durch die Vene 12 ungehindert möglich ist und die Manschette 16 einfach zu entfernen ist. Stattdessen oder zusätzlich zu einer solchen Eingabe durch den Nutzer ist es auch möglich, dass das Steuergerät nach einem bestimmten Zeitablauf automatisch das Ventil 32 öffnet. Damit kann vermieden werden, dass der Blutstau unnötig lange anhält und für die zu behandelnde Person zu gesundheitlichen Risiken führt.

Die bevorzugte und anhand der Figuren 1 und 2 beschriebene Ausführungsform ist stark automatisiert und daher einfach zu bedienen. Damit wird die Gefahr einer Fehlbedienung weitestgehend vermieden, so dass auch ungeübte oder nur gering geschulte Personen damit umgehen können. Das ist insbesondere dann wichtig, wenn eine Person seine eigenen Venen punktieren muss.

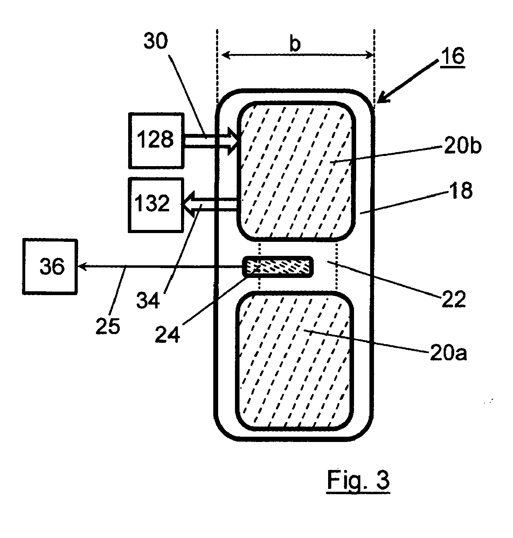

Eine vereinfachte Ausführungsform ist in Fig. 3 gezeigt. Dabei ist die ansteuerbare Pumpe ersetzt durch einen Blasbalg 128, wie er üblicherweise auch bei Armmanschetten zur Blutdruckmessung verwendet wird. Auch das ansteuerbare Ventil wurde ersetzt und zwar durch ein Ventil 132, das manuell betätigt werden kann, ähnlich wie bei einer Armmanschette zur Blutdruckmessung. Wesentlich ist jedoch weiterhin der Sensor 24, der detektieren kann, wann die Blutzufuhr über die Arterie 14 und die Vene 12 erfolgt und wann diese abgeklemmt sind. Der Sensor 24 steuert entsprechend die Anzeigeeinheit 36 an, die entsprechende optische und/oder akustische Signale abgeben kann. Sie unterscheidet sich hier von der aus Fig. 2 im Wesentlichen dadurch, dass sie direkt die Signale des Sensors 24 verarbeiten kann.

Die dargestellten Ausführungsbeispiele lassen sich in unterschiedlichster Weise variieren. Dafür ist insbesondere denkbar:
- Die Anzeigeeinheit 36 ist in dem Sensor 24 integriert.
- Zumindest einzelne der Signale 25, 27, 31, 35 werden drahtlos übertragen, wie über Funk (Bluetooth, WLAN, usw.), Infrarot oder dergleichen. Die Mittel zur Ansteuerung insbesondere der Pumpe 28, des Ventils 32 und/oder der Anzeigeeinheit 36, sowie die elektrischen Versorgungen sind entsprechend anzupassen. Dafür können geeignete Batterien, Akkus oder andere Mittel, wie Induktionsschleifen oder dergleichen verwendet werden.

### Referenzzeichenliste

- 10: Arm
- 12: Vene
- 14: Arterie
- 16: Manschette
- 18: Kompressionsband
- 20a, 20b: erste, zweite Luftkammer
- 22: Übergangskanal
- 24: Sensor
- 25: Sensorsignal
- 26: Elektronisches Steuergerät
- 27: Erstes Steuersignal (zur Pumpenansteuerung)
- 28: Pumpe
- 30: Erster Luftschlauch (von der Pumpe)
- 31: Zweites Steuersignal (zur Ventilsteuerung)
- 32: Automatisches Ventil
- 34: Zweiter Luftschlauch (zum Ventil)
- 35: Anzeigesignal
- 36: Anzeigeeinheit
- 38: Eingabestufe
- 128: Blasbalg
- 132: Manuell betätigbares Ventil

## Patentansprüche

1. Gerät zum Stauen von Blut in einer Vene mit einer Manschette (16), die an einem Körperteil (10) befestigt werden kann, in dem sich eine Vene (12) und eine Arterie (14) befinden, wobei die Manschette (16) eine Kompressionskammer (20) aufweist, die Druck derart auf den Körperteil (10) ausüben kann, dass die Vene (12) und/oder die Arterie (14) verschlossen werden, **dadurch gekennzeichnet, dass** ein Sensor (24) enthalten ist, der bei Befestigung der Manschette (16) an dem Körperteil (10) in direktem oder indirektem Kontakt mit dem Körperteil (10) ist und der so ausgebildet ist, dass er einen Blutfluss durch die Arterie (14) detektieren kann und ein Sensorsignal (25) abgibt, dessen Wert abhängig ist von einem derartigen Blutfluss.

2. Gerät nach Anspruch 1, **dadurch gekennzeichnet, dass** der Sensor (24) als Drucksensor, als Dehnungssensor und/oder als akustischer Sensor ausgebildet ist.

3. Gerät nach Anspruch 1 oder 2, **dadurch gekennzeichnet, dass** die Kompressionskammer (20) derart gestaltet ist, dass sie Druck auf den Körperteil (10) durch Volumenänderung ausübt wobei diese erfolgt durch Zufuhr von Gas und/oder Flüssigkeit und dass die Menge des zugeführten Gases und/oder der zugeführten Flüssigkeit abhängt von der Ansteuerung oder Betätigung einer Zuführeinheit (28, 128).

4. Gerät nach einem der vorigen Ansprüche, **dadurch gekennzeichnet, dass** die Kompressionskammer (20) derart gestaltet ist, dass sie Druck auf den Körperteil (10) durch Volumenänderung ausübt wobei diese erfolgt durch Ablassen von Gas und/oder Flüssigkeit und dass die Menge des abgelassenen Gases und/oder der abgelassenen Flüssigkeit abhängt von der Ansteuerung oder Betätigung einer Ablasseinheit (32, 132).

5. Gerät nach einem der vorigen Ansprüche, **dadurch gekennzeichnet, dass** das Sensorsignal (25) eine Anzeigeeinheit (36) ansteuert, die in Abhängigkeit vom Wert des Sensorsignals (25) ein optisches und/oder akustisches Signal abgibt.

6. Gerät nach einem der vorigen Ansprüche, **dadurch gekennzeichnet, dass** eine Steuereinheit (26) vorhanden ist, die
- nach Empfang eines Start-Signals zunächst ein erstes Steuersignal (27) abgibt, wodurch mittels der Kompressionskammer (20) Druck derart auf den Körperteil (10) ausgeübt wird, dass sowohl der Blutfluss in der Vene (12) als auch in der Arterie (14) vollständig oder nahezu vollständig blockiert wird,
- anschließend ein zweites Steuersignal (31) abgibt, wodurch mittels der Druckkammer (20) der Druck zeitlich gesteuert vermindert wird bis der Wert des Sensorsignals (25) signalisiert, dass Blut durch die Arterie (14) fließt und die
- daraufhin ein entsprechendes Signal (35) an die Anzeigeeinheit (36) abgibt.

7. Gerät nach Anspruch 6, **dadurch gekennzeichnet, dass** die Steuereinheit (26) nach einer bestimmten Zeit durch Abgabe des zweiten Signals (31) die Druckkammer (20) derart ansteuert, dass der durch die Kompressionskammer (20) verursachte Druck auf ein Minimum reduziert wird, so dass ein ungehinderter Blutfluss sowohl durch die Arterie (14) als auch durch die Vene (12) ermöglicht ist.

8. Gerät nach Anspruch 6 oder 7, **dadurch gekennzeichnet, dass** die Steuereinheit (26) nach Eingabe eines Signals durch einen Nutzer durch Abgabe des zweiten Signals (31) die Druckkammer (20) derart ansteuert, dass der durch die Kompressionskammer (20) verursachte Druck auf ein Minimum reduziert wird, so dass ein ungehinderter Blutfluss sowohl durch die Arterie (14) als auch durch die Vene (12) ermöglicht ist.
